# EUROPEAN PATENT SPECIFICATION

(11) **EP 0 819 920 B1**
(45) Date of publication and mention of the grant of the patent: **12.12.2001**
(21) Application number: 96115788.0
(22) Date of filing: 02.10.1996
(51) Int. Cl.: G01G 19/387, G01G 15/02

(54) **Method and apparatus for selecting eggs**
Verfahren und Vorrichtung zur Auswahl von Eiern
Méthode et appareil pour la sélection des oeufs

(30) Priority: 16.07.1996 KR 9628736
(43) Date of publication of application: 21.01.1998
(73) Proprietor: KYOWA MACHINERY CO., LTD., Tsuyama-shi, Okayama-ken (JP)
(72) Inventor: Kondo, Hayashi, Okayama-ken (JP)
(74) Representative: Petersen, Frank, Dipl.-Ing.

(56) References cited:
- GB-A- 2 067 863
- US-A- 3 898 435
- US-A- 4 765 124

## Description

### BACKGROUND OF THE INVENTION

### 1 FIELD OF THE INVENTION

The present invention relates to a method and an apparatus for selecting a group of eggs from a plurality of eggs which are individually weighed in advance and are being conveyed in a row, in such a manner that the individual weight of each selected egg may fit within a predetermined permissible unit weight. range and also that the total weight of the group of selected eggs may fit within a predetermined permissible sum total weight range including a target sum total weight.

### 2. DESCRIPTION OF THE RELATED ART

As a method of packing a group of eggs into a packaging container, there exists the standard method of classifying eggs according to the weight into the size groups of LL, L, M, S and so on and then packing a predetermined number of eggs of a same size group into one container. One problem with this standard packing method arises from the fact that there is general preference among the consumers for the L-size eggs or M-size eggs. This consumer preference leads to disadvantageous increase in the unit cost per unit weight of such preferred eggs over the un-preferred eggs.

Then, in an attempt to level off such difference in the unit cost, the industry has provided standardization of the number of eggs to be packed into one container, a permissible range of the weight of each egg, i.e. the predetermined permissible unit weight of each egg, and the permissible range of the target sum total weight, i.e. the permissible sum total weight range. With the introduction of this standardization, the industry is hoping that the eggs may be packed regardless of their sizes and consequently the new method will allow commercial packaging of a greater number of eggs including those which would be eliminated according to the size-based old method. This new method will be referred to as a 'constant weight packaging method' hereinafter.

As a method of selecting a group of eggs which may be packed by the above-described constant weight packaging method from a plurality of eggs which are being conveyed in a row, there has been suggested one method as described in the Japanese published patent gazette No. 2-29970. According to this method, in selecting any egg other than the last one to be packed into a single container, determination as to whether this egg should be selected or not is made based on whether a 'subtotal' weight which would result from addition of the weight of this egg to the total weight of those foregoing eggs which have already been selected may fit within a predetermined permissible 'subtotal' weight range or not. And, in selecting the last egg to be packed into the same container, the determination as to whether this particular egg should be selected out as the last egg or not is made based on whether or not a 'sum total' weight which would result from addition of the weight of this egg as the last member to the total weight of all the other selected eggs may fit within a predetermined permissible 'sum total' weight range including a target sum total weight.

According to the above-described conventional method, however, if the determination of selection or non-selection from candidate eggs conveyed in a row is made in a strict manner based on a narrowly set subtotal weight range, it will be possible to select out the predetermined number of eggs to be packed into a single package with some degree of certainty. However, because of the strict determination condition, the ratio of the eggs which can satisfy this condition and can thus be selected from the candidate eggs will be low disadvantageously.

Conversely, if the determination is made based on a lax condition, this will result in significant difficulty in the selection of the last egg to be packed into the container, because few of the conveyed eggs will be able to satisfy the requirement of fitting within the predetermined permissible sum total weight range including the target sum total weight. As a result, a situation may occur in which the system keeps running in vain for an extended period of time failing to find the last egg member which can satisfy the above requirement. Or, when the last egg is finally found, a considerable number of eggs will have passed un-selected for packaging. Therefore, in this case too, the ratio of the eggs eligible for the selection for packaging will be eventually low.

The above-described drawbacks of the conventional method are addressed to by the present invention. A first object of the present invention is to provide a method and an apparatus which allows selection of a higher ratio of eggs as being eligible for the constant weight packaging from a plurality of eggs which are being conveyed in a row.

A second object of the invention is to enable the method and apparatus to effect smoothly the egg selection for the constant weight packaging while fully achieving the first object.

### SUMMARY OF THE INVENTION

For accomplishing the above object, a method of selecting eggs, according to the present invention comprises the step of:
selecting a predetermined target number of eggs from a plurality of eggs which are individually weighed in advance and are being conveyed in a row, in such a manner that the individual weight of each selected egg may fit within a predetermined permissible unit weight range and also that the total weight of the group of selected eggs may fit within a predetermined permissible sum total weight range including a target sum total weight;
wherein, until the number of selected eggs reaches a set number, an egg is selected as one of the predetermined target number of eggs if the weight of the egg fits within the predetermined permissible unit weight range; and
after the number of selected eggs has reached said set number, a further egg is selected as another one of the predetermined target number of eggs if the weight of the further egg falls within said predetermined permissible unit weight range and if a subtotal weight resulting from addition of the weight of the further egg to the total weight of the previously selected eggs falls within a selective permissible weight range which becomes narrower with increase in the number of selected eggs.

With the above construction, in selecting the first egg to the last one within the set number of eggs, no special selection condition is set except that of the egg weight having to be within the predetermined permissible unit weight range. Hence, the efficiency of selecting the eggs eligible for the constant weight packaging may be improved. Moreover, it is experimentally known that there exists normal distribution in the weights of the eggs which are conveyed in a row. Thus, the sum total weight of the eggs selected for the constant-weight packing will not significantly vary.

On the other hand, after the number of the selected eggs has exceeded the set number, the selection of a further egg for the constant-weight packing is made, based not only on the above condition of the weight of the further egg having to be within the predetermined permissible unit weight, but also on the further condition of a subtotal weight resulting from addition of the weight of the further egg to the total weight of all the previously selected eggs falls within a selective permissible weight range which becomes narrower with increase in the number of selected eggs.

Namely, as the selective permissible weight range is variable to become narrower instead of being constant, the added-up or sub-total weight of the eggs selected for the constant-weight packaging may smoothly converge at the target sum total weight in a reliable manner.

Accordingly, according to the method of the invention, while utilizing the experimental finding of normal distribution being present in the weight of the selected eggs, the selecting efficiency in selecting those eggs eligible for the constant-weight packaging may be improved and also the selecting condition is set in such a manner to allow the added-up or subtotal weight of the eggs selected for the constant-weight packaging to converge at the target sum total weight in a reliable manner.

As a result, the invention has achieved the object of providing a method and an apparatus which allows selection of a higher ratio of eggs as being eligible for the constant weight packaging from a plurality of eggs which are being conveyed in a row.

Preferably, with selection of a further egg, a lower limit value of said selective permissible weight range increments, from an expected minimum added-up weight of the set number of selected eggs, by a value obtained by dividing a difference between a lower limit value of the target sum total weight range and the expected minimum added-up weight of the set number of selected eggs by a difference between the target number and the set number. Similarly, with selection of the further egg, an upper limit value of said selective permissible weight range increments, from an expected maximum added-up weight of the set number of selected eggs, by a value obtained by dividing a difference between an upper limit value of the target sum total weight range and the expected maximum added-up weight of the set number of selected eggs by a difference between the target number and the set number.

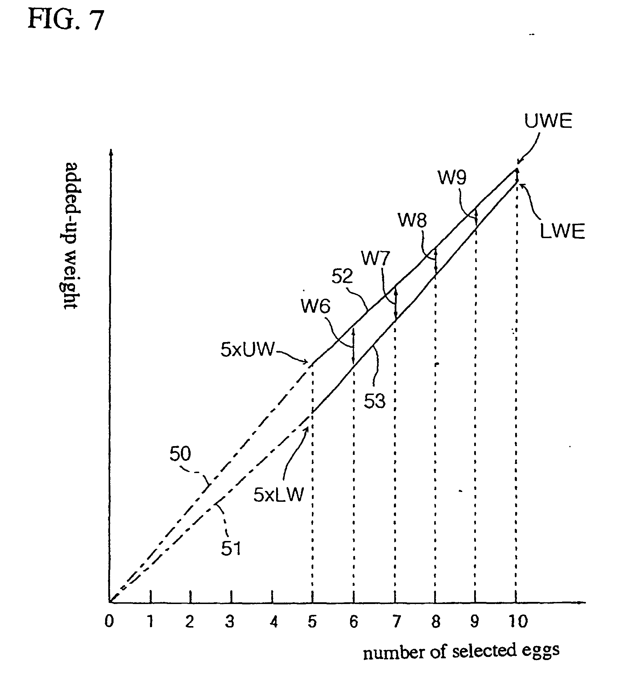

To describe the above more specifically, a graph may be drawn to cause the horizontal axis thereof to represent the number of eggs selected for the constant-weight packaging and to cause the vertical axis thereof to represent the added-up weight of these eggs. In this graph (see Fig. 7), the lower limits of above-described selective permissible weight range are represented by a straight line extending from the expected minimum added-up weight of the set number of eggs and the lower limit value of the predetermined permissible weight range. Whereas, the upper limits of the selective permissible weight range are represented by a further straight line extending from the maximum added-up weight of the set number of eggs and the upper limit value of the predetermined permissible weight range.

That is to say, the selective permissible weight range is adapted to decrease in the (vertical) width thereof as approaching the target number, and the rate of this reduction in the width is set to be constant in order to avoid extreme and abrupt change in the degree of selection stringency.

As a result, the selection of eggs for the constant-weight packaging may be effected smoothly.

Preferably, the set number is set substantially to the half of the target number.

Namely, substantially a half of the target number, i.e. total number of eggs for one constant-weight packaging are set as the set number of eggs. So that, each of about half the target number of eggs may be selected based on the sole requirement of its weight being within the predetermined permissible unit weight. Each of the further half of the eggs will be selected based also on the other additional requirement of its weight being within the selective permissible weight range.

Accordingly, the portion selected based on the relatively lax selection condition and the other portion selected based on the more stringent selection condition may be well balanced with each other. As a result, the selection of eggs for the constant-weight packaging may be effected still more efficiently.

Further and other objects, features and effects of the invention will become more apparent from the following more detailed description of the embodiments of the invention with reference to the accompanying drawings.

### BRIEF DESCRIPTION OF THE DRAWINGS

Fig. 1 is a plan view showing a schematic construction of an egg selecting-packaging apparatus relating to one preferred embodiment of the present invention,
Fig. 2 is a side view showing the schematic construction of the egg selecting-packaging apparatus of the embodiment,
Fig. 3 is a block diagram of a control construction relating to the embodiment.
Fig. 4 is an enlarged view of major portions relating to the embodiment,
Fig. 5 is a further enlarged view of major portions relating to the embodiment,
Fig. 6 is a still further enlarged view of major portions relative to the embodiment,
Fig. 7 is a graph illustrating an egg selecting scheme relating to the embodiment of the invention,
Figs. 8(a), 8(b) and 8(c) are a series of flow chart relating to the embodiment of the invention,
Fig. 9 is a further flow chart relating to the embodiment of the invention,
Figs. 10(a) and 10(b) are a series of further flow chart relating to the embodiment,
Fig. 11 is a further flow chart relating to the embodiment,
Fig. 12 is a further flow chart relating to the embodiment, and
Fig. 13 is a descriptive view of a kick data look-up table relating to the embodiment of the invention.

### DETAILED DESCRIPTION OF THE PREFERRED EMBODIMENTS

An egg selecting-packaging apparatus relating to one preferred embodiment of the invention will be described next with reference to the accompanying drawings.

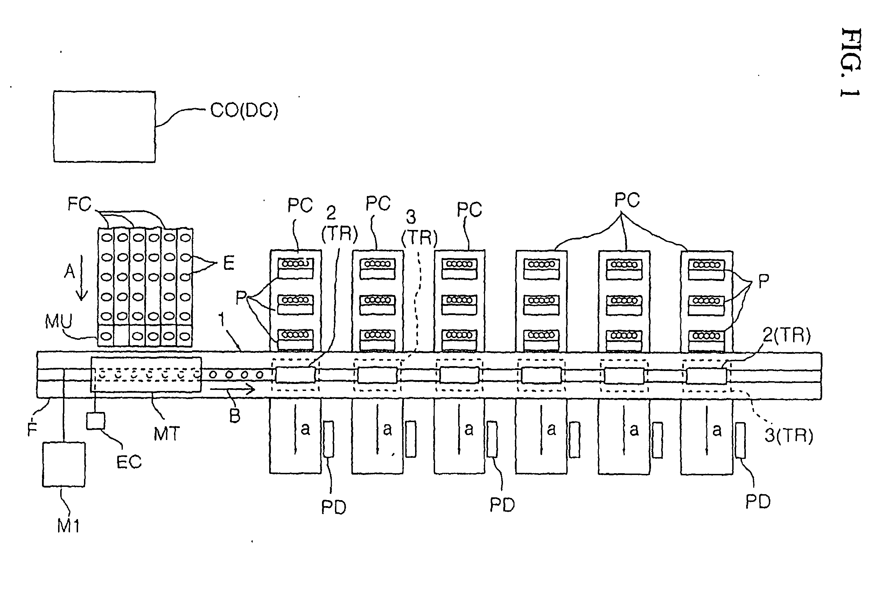

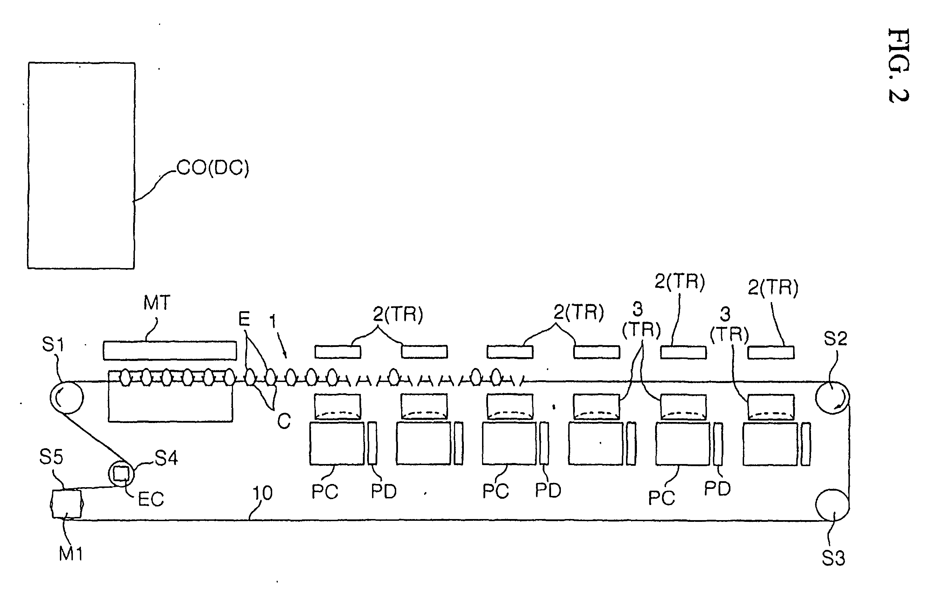

The egg selecting-packaging apparatus shown in the plan view of Fig. 1 as well as in the side view of Fig. 2 includes six rows of egg feeding conveyers FC for conveying eggs in a direction of an arrow A, a single-row conveyer 1 for receiving eggs from the egg feeding conveyers FC and then conveying the eggs in a direction of an arrow B, a plurality of package conveyers PC for conveying packaging containers P in a direction of an arrow (a) normal to the conveying direction of the single-row conveyer 1, and a control device CO for controlling various operations of the respective conveyers.

The eggs E fed from the egg feeding conveyers FC are weighed individually and then transferred on to the single-row conveyer 1 and conveyed to a position where the single-row conveyer 1 runs across the package conveyer PC. In the course of this, the control device CO determines, based on the individually measured weights of the eggs E, whether these eggs may be selected for the constant-weight packaging or not. When those eggs selected for the constant-weight packaging reach the container conveyer PC which is designated for the constant-weight packaging, the eggs are transferred into a packaging container P conveyed by this package container PC.

The other eggs E which were not selected for the constant-weight packaging are conveyed further to a position where the single-row conveyer runs across a package conveyer PC other than the one conveyer PC designated for the constant-weight packaging and then these eggs are transferred into a packaging container P by e.g. the size-based packaging method according to the Japanese Agricultural Standard (JAS).

Each packaging container P after completion of the packaging operation of the eggs E therein is taken out as a final product from a downstream end of the conveying direction of the container P by the package conveyer PC.

Next, the respective components will be described in details.

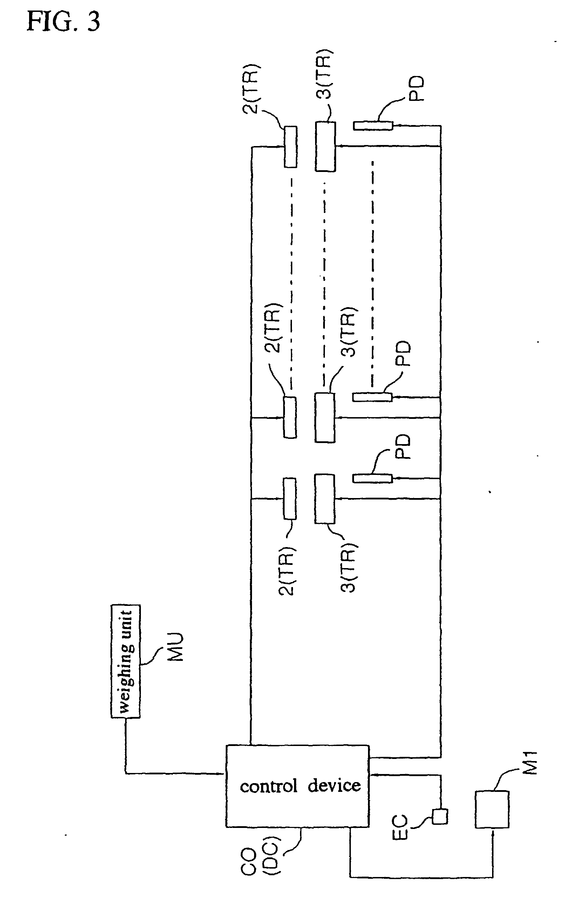

The egg feeding conveyer FC conveys the eggs E with an appropriate space therebetween. At an end of each egg feeding conveyer FC adjacent the single-row conveyer 1, there is provided a weighing unit MU for individually weighing the egg E conveyed from the egg feeding conveyer FC. The weight datum obtained by the weighing unit MU about the egg E is transferred to the control device CO, as illustrated in a block diagram of Fig. 3.

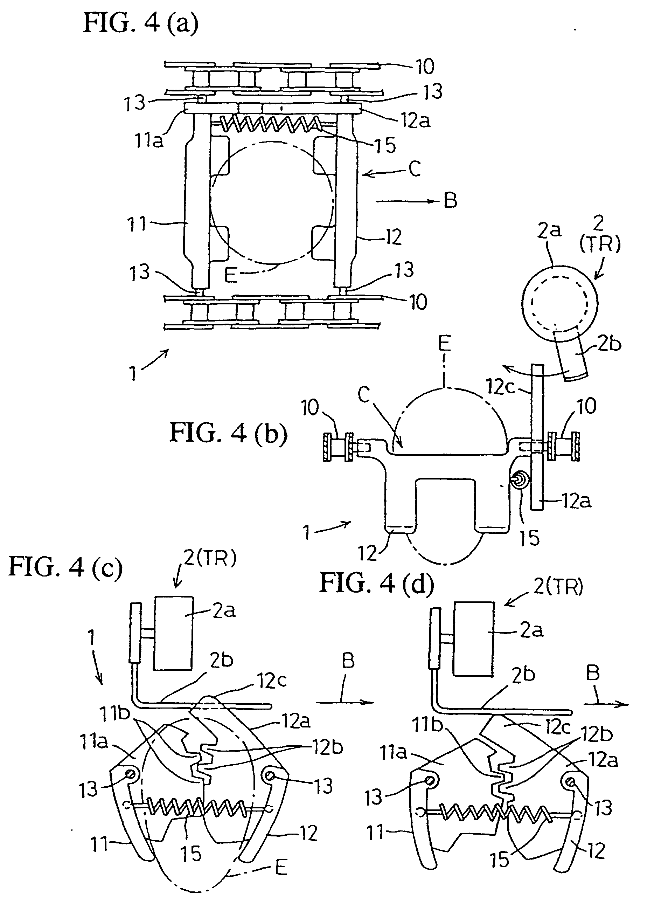

The single-row conveyer 1 includes a pair of right and left chains 10 which hold right and left ends of a plurality of cups C each holding an egg E thereon as shown in Fig. 4, driven sprockets S1, S2, S3 and S4 about which the chains 10 are entrained, a drive motor M1 for rotatably driving the chains 10 through drive sprockets S5, a transfer device MT for transferring the eggs E from the weighing units MU of the egg feeding conveyers FC on to the cups C, a plurality of kicking devices 2 for causing the eggs E to be dropped off the cups C, and a plurality of packing devices 3 for receiving the eggs E dropped off the cups C by the operations of the kicking devices 2 and then packing these eggs E into the packaging containers P conveyed by the packaging container conveyers PC. And, these components are mounted and assembled together on a frame structure.

As illustrated in the block diagram of Fig. 3, the drive motor M1 is controlled by the control device CO. Specifically, the feeding amount of the chains 10, i.e. the conveying amounts of the cups C are detected by an encoder EC attached to the driven sprocket S4 and then the data are transferred to the control device CO. Incidentally, the driving power of the driving motor M1 is taken off also to the egg feeding conveyers FC.

As shown in Fig. 4, each cup C includes a rear support member 11 and a front support member 12 which members are attached via pins 13 to the pair of right and left chains 10 to be pivotable about an axis extending in the right-to-left direction. The rear support member 11 and the front support member 12 respectively has a holding portion for holding an egg E which portion presents a substantially hook-like shape in a plan view and extends obliquely downwards. Such that, these obliquely downwardly extending holding portions of the support members 11 and 12 extend from the opposite directions toward the center of the egg E so as to follow the contour of the egg E.

The rear support member 11 forms a rear side plate 11a at the left end thereof. Similarly, the front support member 12 forms a front side plate 12a at the left end thereof. These rear and front side plates 11a, 12a are respectively connected with opposite ends of a spring 15, such that the spring 15 urges lower ends of these plates 11a, 12a to approach each other. The rear side plate 11a includes, adjacent a leading end thereof, a projecting portion 11b which is formed like a toothed wheel centered about the rear pin 13. Similarly, the front aide plate 12a, includes, adjacent a leading end thereof, a projecting portion 12b which is formed like a toothed wheel centered bout the front pin 13. And, these projecting portions 11b and 12b are in contact with each other.

The front support member further includes a projecting element 12c which projects obliquely upwardly. Then, as will be described later, when this projecting element 12c is pushed by the kicking device 2, the rear support member 11 and the front support member 12 are pivoted about the respective axes.

The kicking device 2 includes five pairs of rotary solenoids 2a and kicking arms 2b shown in Figs. 4(b), (c) and (d) arranged one after another along the conveying direction denoted with the arrow B of the eggs E by the single-row conveyer 1. The operations of these rotary solenoids 2a of the kicking device 2 are controlled by the control device CO, as illustrated in the control block diagram of Fig. 3.

As shown in Figs. 4(c) and (d), the kicking arm 2b has a substantially L-shaped configuration and is pivoted in a direction of an arrow in Fig. 4(b). With this pivotal movement in the direction of the arrow, the kicking arm 2b is moved into a moving path of the projecting element 12c of the cup C; and when the cup C is advanced to the position of this kicking device 2, the kicking arm 2b and the projecting element 12c come into contact against each other. With this contact, the projecting element 12c is pushed and the front support member 12 is pivoted about the pin 13 counter-clockwise in Fig. 4(d). This pivotal movement of the front support member 12 is transmitted via the gear-teeth-like projecting portions 11b, 12b to the rear support member 11, so that the rear support member 11 is pivoted about the pin 13 clockwise in Fig. 4(d).

Consequently, if an egg E is held in this cup C, the egg E is dropped down.

On the other hand, if the kicking arm 2b is at the position shown in Fig. 4(b), the egg E of the cup C just passes by without being dropped, as illustrated in Fig. 4(c).

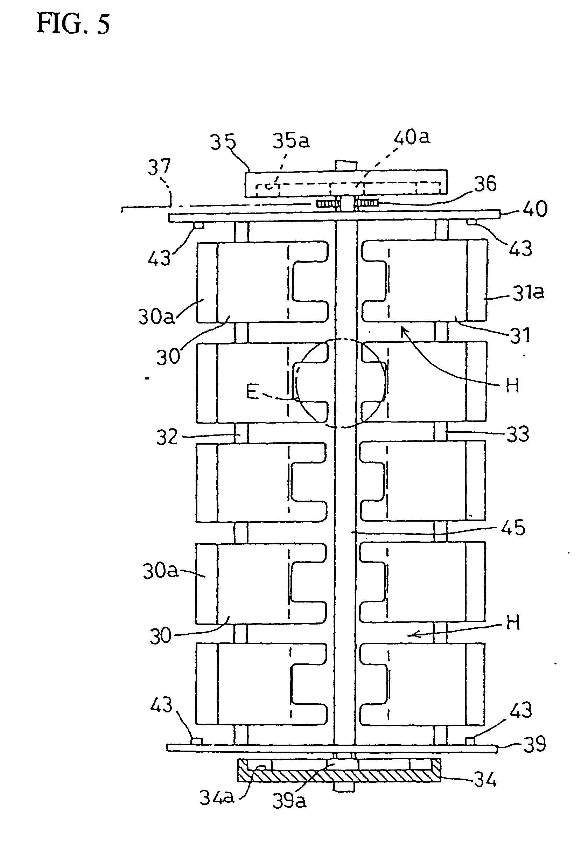

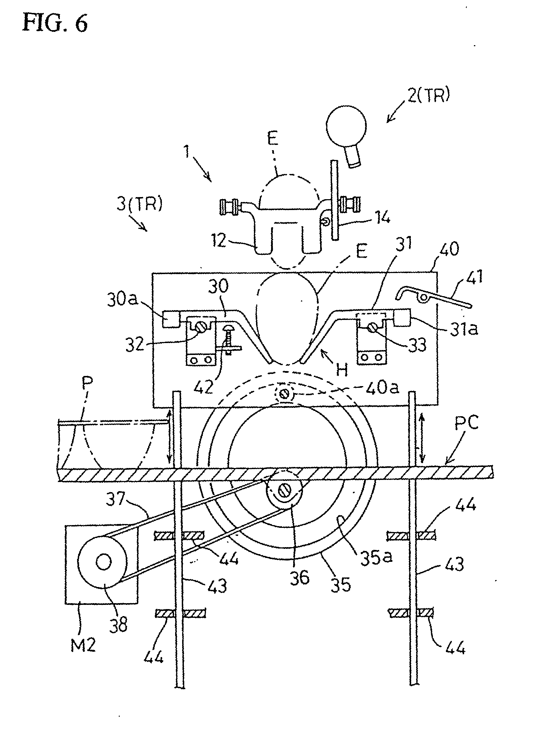

The packing device 3 is disposed downwardly of the kicking device 2. More particularly, as shown in the plan view of Fig. 5 as well as in the side view of Fig. 6, the packing device 3 consists mainly of five holders H for receiving the eggs E dropped from the cups C, a pair of shafts 32, 33 for pivotably supporting the holders H, a pair of plates 39, 40 for fixedly supporting the shafts 32, 33, four guide arms 43 extending downwardly from four corners of the plates 39, 40, guides 44 for slidably guiding the four guide arms 43 through upper and lower guide holes, a pair of eccentric cams 34, 35 for coming into engagement with cam followers 39a, 40a provided at the respective bottom portions of the pair of plates 39, 40, a cam shaft 45 for fixedly and operatively interconnecting the pair of eccentric cams 34, 35 for operating these cams 34, 35 in association with each other, and a drive motor M2 for rotatably driving the cam shaft 45.

Incidentally, in the instant embodiment, the packaging container P is a 10-unit package type having two egg receiving rows each adapted for receiving 5 (five) eggs in total. Instead, in the present invention, a different type of packaging container p may be employed such as one in which 6 (six) eggs are stored in each row. In such case, the number of the rotary solenoids 2a, kicking arms 2b and the holders H will vary in accordance with the number of eggs to be accommodated in a single row.

Each holder H includes a pair of right and left support arms 30, 31 having a substantially hook-shaped egg holding end, a pair of balance weights 30a, 31a attached to the opposite ends of the respective support arms 30, 31 relative to the hooked-shaped egg holding ends across the support shafts 30, 31, a stopper 42 for limiting the clockwise pivotal movement in Fig. 6 of the one support arm 30, and a locking device 41 for regulating the counter-clockwise pivotal movement in Fig. 6 of the other support arm 31.

When the holders H hold no eggs E, by the function of the balance weights 30a, 31a the right and left support arms 30, 31 are maintained still at a stationary posture without contacting stopper 42 and the locking device 41 respectively. Whereas, when the eggs E are dropped from the cups C on to the holders H, the right and left arms 30, 31 are shifted in their posture until they come into contact with the stopper 42 and the locking device 41 respectively. With this shifting in the posture, the dropping impact of the eggs E may be effectively absorbed.

With an instruction from the control device CO, the drive motor M2 rotatably drives the eccentric cams 34, 35 in unison through a drive gear 38 fitted on a rotary shaft of the drive motor M2, a driven gear 36 fitted on a cam shaft 45, and a drive chain 37 entrained between and about the drive gear 38 and the driven gear 36.

The eccentric cams 34, 35, as shown in Fig. 6, each has a substantially circular disc-like configuration and define cam grooves 34a, 35a concentric with the outer peripheral edge adjacent the outer periphery thereof. And, these cam grooves 34a, 35a engage and guide the cam followers 39a, 40a attached to the respective bottom portions of the plates 39, 40.

The cam shaft 45, as shown in Fig. 6, extends through the eccentric cams 34, 35 at a position off the center of the circles of the eccentric cams 34, 35. Therefore, when the drive motor M2 rotatably drives this cam shaft 45, the cam followers 39a, 40a are engaged with and guided by the cam grooves 34a, 35a, whereby the plates 39, 40 are moved up and down.

When the vertically moving plates 39, 40 are at the bottom position, namely, when the holder H is located at its bottom position, the locking device 41 is released to allow the egg E held in the holder H to be dropped into the packaging container P having been conveyed by the package conveyer PC to the position under the holder H.

As shown in Fig. 1, the package conveyer PC conveys each packaging container P with a lid thereof opened, by means of a package conveyer drive unit PD disposed at a downstream position of conveying the package containers P.

As shown in the control block diagram of Fig. 3, the package conveyer drive unit PD is controlled by the control device CO so as to effect the conveying operation of the package containers P in synchronism with the packing device 3.

Next, with reference to Fig. 7, there will be described a method of the invention for selecting or not selecting eggs for the constant-weight packaging based on the weights of the eggs individually measured by the weighing unit MU. Incidentally, in the graph of Fig. 7, the horizontal axis represents the number of eggs selected for the constant-weight packaging and the vertical axis represents the added-up or sub-total weight of these eggs selected for the constant-weight packaging, respectively.

In this selection method according to the invention, from the first member egg to the last member egg within a set number of eggs, the determination of selection or non-selection for the constant-weight packaging is made based solely on whether the weight of each egg falls within a predetermined permissible unit weight range or not. In the instant embodiment, the predetermined target sum total number of eggs to be packed together in one constant-weight packaging is 10 (ten), and the above-mentioned set number is 5 (five).

If eggs are selected based on the above condition, the maximum range of dispersion of the added-up weight of the selected eggs may be defined by dashed lines 50, 51 in Fig. 7. If the lower limit value of the predetermined permissible unit weight range is LW and its upper limit value is UW, when five eggs have been selected, the dashed line 50 in Fig. 7 presents a value: 5 x UW, and the other dashed line 51 presents a value: 5 x LW.

Then, in subsequently selecting the sixth member to the tenth member, selection is made based not only on the above-described condition defined by the predetermined permissible unit weight range, but also on a further condition as follows. Specifically, in this graph of Fig. 7, the lower limit value of a predetermined permissible sum total weight range, i.e. the total weight of the constant-weight packaging is represented by a point denoted with a mark LWE and its upper limit value is represented by a point denoted with a mark UWE, Then, an upper straight line 52 is drawn between the point: 5 x UW and the point UWE, and a lower straight line 53 is drawn between the point: 5 x LW and the point LWE, respectively. Then, the selection is made based on whether or not a subtotal weight resulting from addition of the weight of a further candidate egg to the added-up weight of all the previously selected eggs may fit within an area defined or surrounded by the the upper straight line 52 and and the lower straight line 53.

That is to say, the selection of the sixth, seventh, eighth or ninth member for the constant-weight packaging is based on whether the above-defined subtotal weight falls within a selective permissible weight range represented by vertical widths between the upper and lower straight lines denoted with marks W6, W7, W8 and W9 respectively.

In the above, the respective lower limit value of the ranges W6, W7, W8 and W9 increments, with each selection of another egg, from the expected minimum added-up weight: 5 x LW of the set number of selected eggs, by an amount corresponding to a value resulting from division of a difference between the lower limit value LWE of the predetermined permissible sum total weight range and the expected minimum added-up weight, i.e. 5 x LW by a difference between the predetermined target number and the set number, which difference is '5'(five) in this case.

Similarly, the respective upper limit value of the ranges W6, W7, W8 and W9 increments, with each selection of another egg, from the expected maximum added-up weight: 5 x UW of the set number of selected eggs, by an amount corresponding to a value resulting from division of a difference between the upper limit value UWE of the predetermined permissible sum total weight range and the expected maximum added-up weight, i.e. 5 x UW by the difference between the predetermined target number and the set number, which difference is '5'(five) in this case.

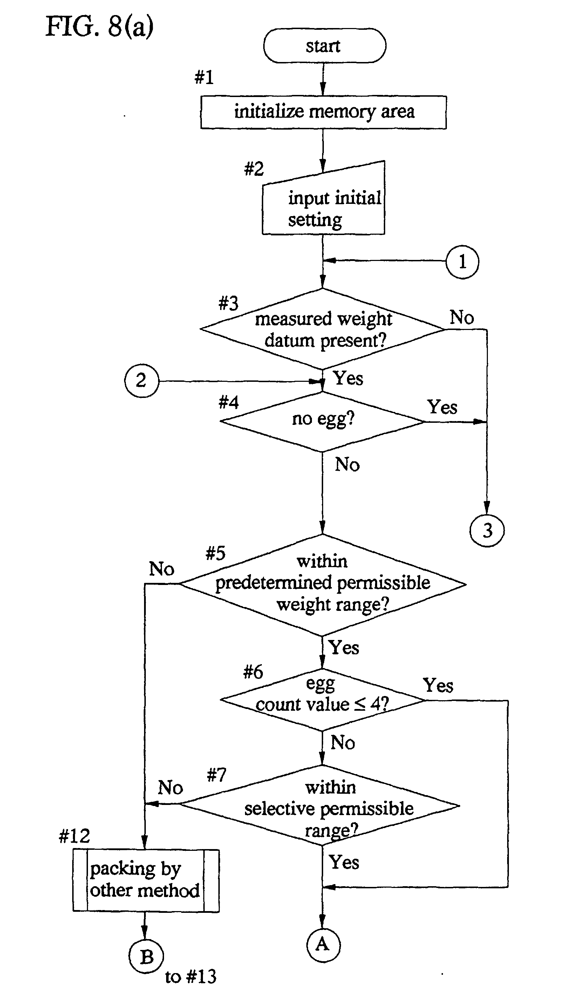

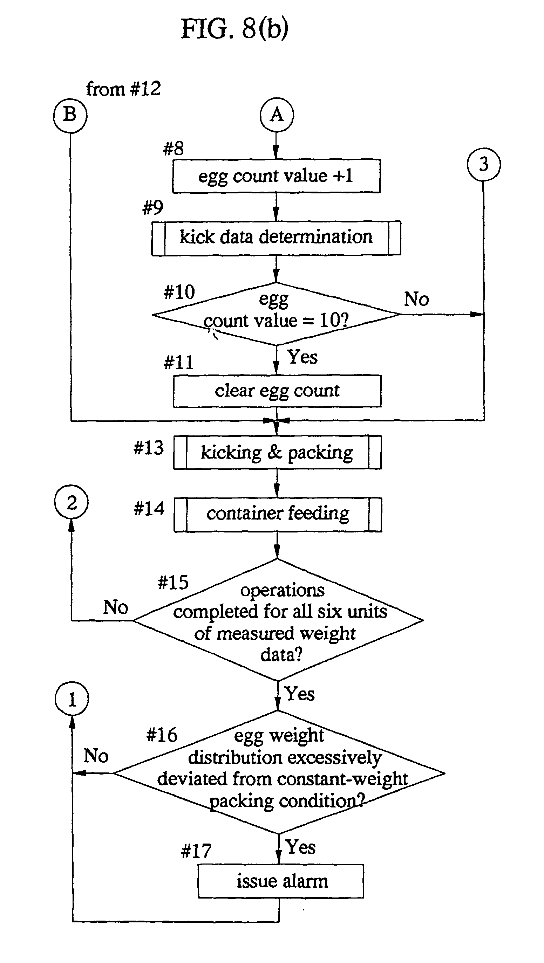

Next, the process of selection and packaging of the eggs E under the control by the control device CO will be described with reference to flow charts shown in Figs. 8 through 12.

With an activation of the selecting-packaging apparatus, memory contents of a storage unit incorporated in the control device CO are initialized (step #1), and the unit is set ready for accepting initial setting inputs (step #2).

The initial setting inputs may include an instruction as to which of the six rows of package conveyers PC is to be designated for the constant-weight packaging of the eggs E, an instruction on the predetermined permissible sum total weight range of the constant-weight packaging, an instruction on the predetermined permissible unit weight range for the constant-weight packaging, and an instruction on a type of packaging method to be effected by the other package conveyers PC not designated for the constant-weight packaging. Incidentally, as described hereinbefore, the number of eggs E to be packed into one packaging container P by the constant-weight packing is set as 10 (ten) in the instant embodiment. Yet, this number may vary depending on the convenience.

After the initial setting, the control device CO inputs a measured weight datum of an egg E from the the weighing unit MU of the egg feed conveyer FC (step #3). Then, comparison is made between a value of the measured weight included in the datum and a predetermined value. If the value of the datum is judged to be greater than the predetermined value thus indicating that the measurement was effected properly with presence of an egg E at the weighing unit MU (step #4), further judgment is made whether the value of the measured weight datum of the egg E is between the upper limit and the lower limit, i.e. whether it is within the predetermined permissible unit weight range (LW to UW) or not (step #5).

If the measured weight of the egg E is within the predetermined permissible unit weight range, the process proceeds to check a count value on an egg counter which counts up the number of eggs up to '10' selected for the constant-weight packaging. If the count value is '4' or less, then, the process just proceeds to select this egg E as a member for the constant-weight packaging. Conversely, if the count value is '5' or more, then, the process further judges whether the subtotal weight is within the selective permissible weight range or not (step #7). If the subtotal weight is within this range, this egg is selected as a further member for the constant-weight packaging.

In either case, with determination of selection of an egg for the constant-weight packaging, the egg number counter is incremented by '+1' (step #8). The process then executes a 'kick data determination process' for determining whether the selected egg E should be dropped into the packing device 3 by the kicking device 2 or not.

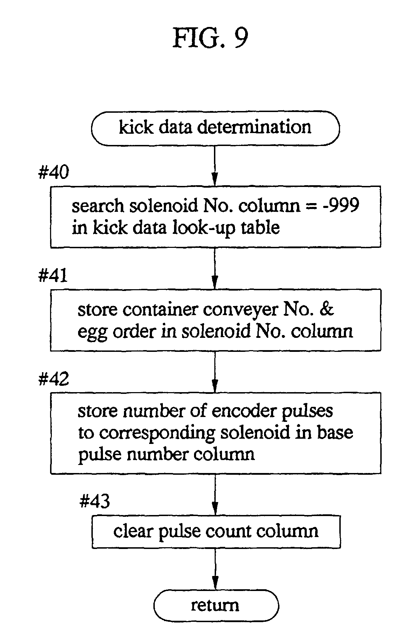

This kick data determination process is effected in accordance with a scheme illustrated by the flow chart of Fig. 9.

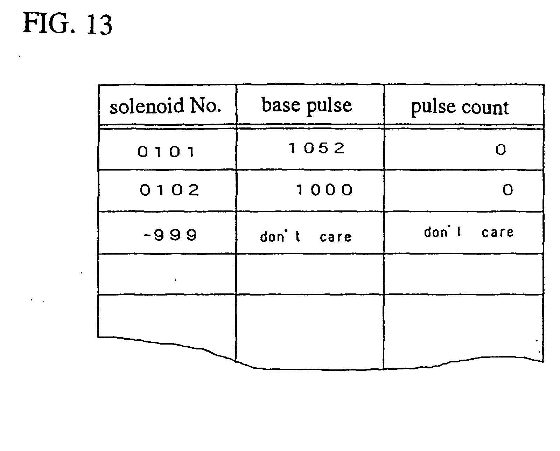

For this kick data determination process, the storage unit incorporated within the control device CO stores therein a 'kick data look-up table' shown in Fig. 13. The kick data look-up table includes three columns in total as follows. Namely, a serial solenoid number table is to be looked up for specifying a rotary solenoid 2a of a kicking device 2 corresponding to a position where the egg E selected for the constant-weight packaging should be dropped off the single-row conveyer 1. A base pulse number column is used for storing a conveying amount from a position of the selected egg E on the single-row conveyer 1 at the time of its selection for the constant-weight packaging to the position of the kicking device 2 specified by the serial solenoid number stored in the serial solenoid number column of the same row, with the conveying amount being converted into a number of pulses of the encoder EC, A pulse count column is used for holding a count value of pulses outputted by the encoder EC with an interrupt executed with each pulse output from the encoder EC. The kick data look-up table includes a number of rows corresponding to the number of cups C present between the sprockets S1, S2 of the single-row conveyer 1.

The serial solenoid number column holds a four-digit figure, the first two digits indicating the serial number of the package container PC and the subsequent two digits indicating the count value of the eggs. That is to say, since the order of the selected egg E is caused to correspond to the order of the five kicking devices 2, a kicking device 2 may be specified by such four-digit figure.

In the kick data determination process, first, the process searches, in the kick data look-up table, a row whose serial solenoid number column stores therein a value '-999' indicating an empty status (step #40), and stores, in this serial solenoid number column, the serial number of the package conveyer PC and the order of the selected egg E (step #41). Then, the process stores, in the base pulse number column belonging in the same row, the number of pulses of the encoder EC to be counted to the position of the particular rotary solenoid 2a, i.e. the corresponding kicking device 2 (step #42) and then clears the pulse count column belonging in the same row.

Thereafter, the number of pulses outputted by the encoder EC will be counted up in this cleared pulse count column.

After completion of the kick data determination process described above, if the egg count value has reached '10' (step #10), this egg count value is cleared (step #11) and the process executes a kicking and packing operation (step #13).

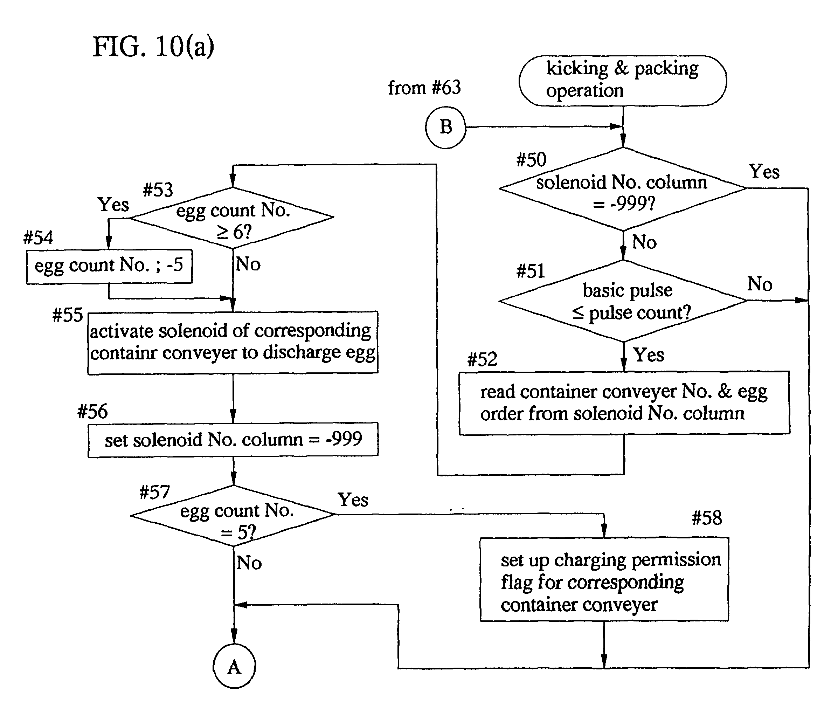

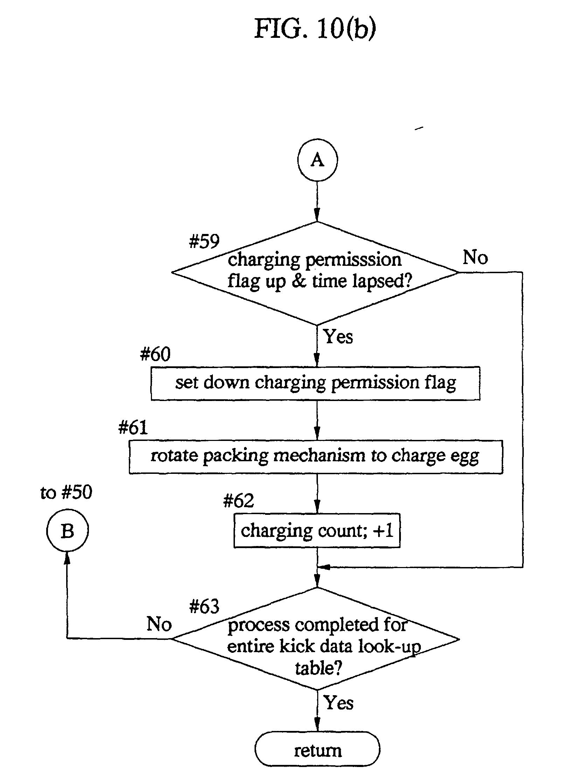

In this kicking-packing operation, in accordance with the flow chart of Fig. 10, the process confirms for each row of the kick data look-up table whether the selected egg E has been conveyed to the target position or not. If so, the egg is allowed to be dropped, thus carrying out the kicking-packing operation.

First, the process searches, from the respective rows of the kick data look-up table, one whose serial solenoid number column stores therein a value other than '-999' (step #50) and also whose base pulse count column stores therein a value smaller than that stored at the pulse count number column indicating that the egg E selected for the constant-weight packaging has been already conveyed to the target position where the egg is to be dropped (step #51).

When such egg having been conveyed to the target position has been found, based on the value stored in the serial solenoid number column, the process reads the serial number of the package conveyer PC and also the current egg count value, i.e. the order of this selected egg E in the group of total 10 eggs (step #52). If the egg count number is 6 or more (step #53), for the correspondence with the serial number of the kicking device 2, '5' is subtracted from the egg count number and the resultant difference is specified as the serial number of the kicking device 2 (step #54). Then, a rotary solenoid 2a corresponding to the serial number of the package conveyer PC and the serial number of the kicking device 2 is activated to discharge this egg E to the packing device 3 (step #55).

After completion of the above operation, the data held in the serial solenoid number column become unnecessary, so that the data '-999' indicating the empty status are stored therein instead (step #56).

In the above, if the egg number, i.e. the egg count value or the difference value obtained by subtracting '5' thereof, when the latter is appropriate, is '5', i.e. when all of the five holders H of the packing device 3 are now holding eggs E (step #57), then, the process sets up a flag for permitting charging of the eggs into a packaging container P (step #58).

If the charging permission flag is set up and a predetermined time period has lapsed (step #59), the charging permission flag is set down (step #60) and the drive motor M2 of the packing device 3 is activated to rotate the eccentric cams 34, 35 for one rotation (step #61). In the course of this one rotation of the eccentric cams 34, 35, when the holders H are located at the bottom end position, the locking device 41 is released to allow the eggs E to be dropped into the packaging container P.

With one rotation of the eccentric cams 34, 35, a charging count adapted for indicating the number of chargings from the packing device 3 to the packaging container P is incremented by '+1' (step #62).

The above-described series of steps are repeated for all of the rows of the kick data look-up table. With completion of the steps for all the rows (step #63), the process returns to the process of the flow chart in Fig. 8(b), and proceeds to carry out a container feeding operation (step #14).

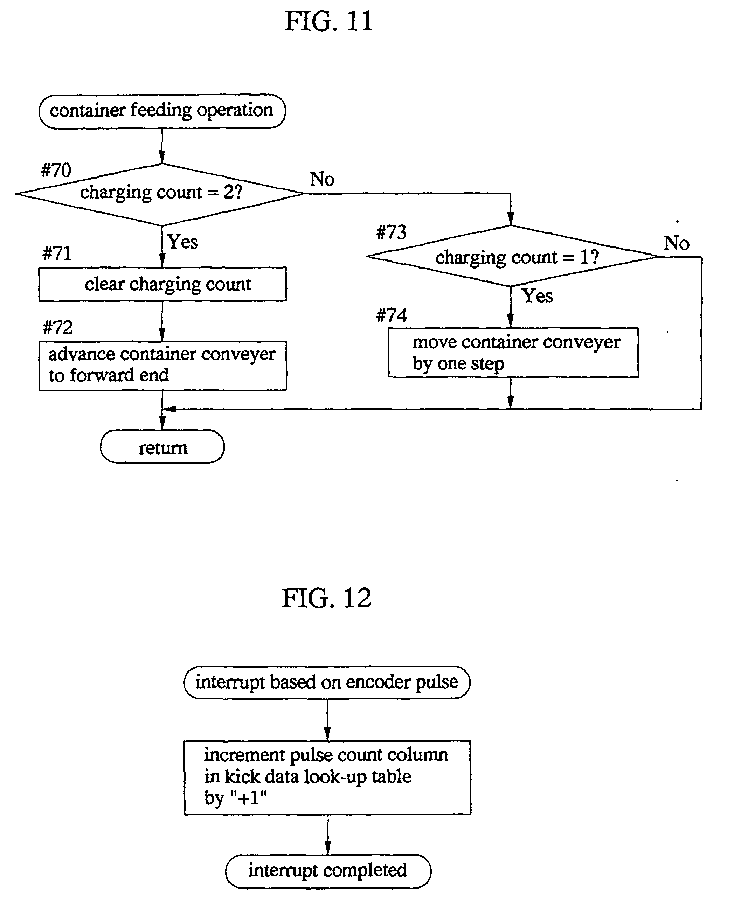

This container feeding operation is carried out in accordance with the flow chart of Fig. 11.

In this container feeding operation, if the charging count value has become '2' indicating completion of storage of all eggs E into one packaging container P (step #70), the process clears this charging count value (step #71) and transports the next packaging container P to the leading position at the bottom of the packing device 3 (step #72).

On the other hand, if the charging count value is '1' indicating completion of storage of only one row of the total two rows in the packaging container P (step #73), this packaging container P is transported by an amount corresponding to the space of one row only (step #74). Incidentally, when the charging count value is '0', no operation is effected at all.

After completion of the feeding operation of the packaging container P, the process returns to the flow chart of Fig. 8.

The above completes the entire process for a single unit of measured weight datum of the egg E received by the control device from the weighing unit MU. Yet, since the weighing unit MU is provided for each of the six rows of egg feeding conveyers FC, the control device receives 6 units of measured weight data at one time.

Accordingly, the process repeatedly carries out the steps subsequent to the confirmation of presence of the egg (step #4) for all of these six units of measured weight data; and when these operations are completed (step #15), the process determines, based on e.g. the average weight value of the eggs, whether or not the distribution of the weight of the eggs excessively deviates from the requirement for the constant-weight packaging, i.e. the predetermined permissible sum total weight range (step #16). If such excessive deviation is found, the process issues an alarm (step #17) and waits for different measured weight data while carrying out the kicking-packing operation (step #13) and the container feeding operation (step #14).

Incidentally, if the weight of the egg E is not within the predetermined permissible unit weight range (step #5) or when the subtotal weight is not within the selective permissible weight range (step #7), then, the constant-weight packaging is not effected. Instead, some other packaging operation such as the size-based packaging method according to the JAS standard will be carried out (step #12)o.

Accordingly, the kicking device 2 and the packing device 3 together function as a transferring means TR for transferring eggs into a packaging container P. The control device CO functions as a determining means DC for determining selection or non-selection of an egg E for the contact-weight packaging.

Some other embodiments will be described next.
(1) In the foregoing embodiment, the set number within which the determination of selection of an egg for the constant-weight packaging is made based solely on whether its weight falls within the predetermined permissible unit weight range is '5' (five). This set number may vary depending on the convenience.
(2) In the foregoing embodiment, the selective permissible weight range is defined by the area surrounded between the straight line 52 extending between the value: 5 x UW and the further value: UWE and the further straight line 53 extending between the value: 5 x LW and the further value: LWE. Instead, provided that the width of this selective permissible weight range decreases with increase in the number of selected eggs, the lines extending between 5 x UW and UWE and between 5 x LW and LWE may not be straight lines.
(3) In the foregoing embodiment, the transferring means TR comprises the kicking device 2 and the packing device 3. Instead, a manipulator or the like may be provided for transferring eggs E conveyed on the single-row conveyer 1 into the packaging container P.

## Claims

1. A method of selecting eggs comprising the step of:
selecting a predetermined target number of eggs from a plurality of eggs which are individually weighed in advance and are being conveyed in a row, in such a manner that the individual weight of each selected egg fits within a predetermined permissible unit weight range and also that the total weight of the group of selected eggs fits within a predetermined permissible sum total weight range. including a target sum total weight;
**characterized in that**
until the number of selected eggs reaches a set number, an egg is selected as one of the predetermined target number of eggs if the weight of the egg fits within the predetermined permissible unit weight range; and
after the number of selected eggs has reached said set number, a further egg is selected as another one of the predetermined target number of eggs if the weight of the further egg falls within said predetermined permissible unit weight range and if a subtotal weight resulting from addition of the weight of the further egg to the total weight of the previously selected eggs falls within a selective permissible weight range which becomes narrower with increase in the number of selected eggs.

2. A method as defined in claim 1,
**characterized in that**
with selection of a further egg, a lower limit value of said selective permissible weight range increments, from an expected minimum added-up weight of the set number of selected eggs, by a value obtained by dividing a difference between a lower limit value of the target sum total weight range and the expected minimum added-up weight of the set number of selected eggs by a difference between the target number and the set number; and
with selection of the further egg, an upper limit value of said selective permissible weight range increments, from an expected maximum added-up weight of the set number of selected eggs, by a value obtained by dividing a difference between an upper limit value of the target sum total weight range and the expected maximum added-up weight of the set number of selected eggs by a difference between the target number and the set number.

3. A method as defined in claim 1 or 2,
**characterized in that**
said set number is set substantially to the half of said target number.

4. An apparatus for selecting eggs, wherein a predetermined target number of eggs from a plurality of eggs which are individually weighed in advance and are being conveyed in a row, in such a manner that the individual weight of each selected egg may fit within a predetermined permissible unit weight range and also that the total weight of the group of selected eggs may fit within a predetermined permissible sum total weight range including a target sum total weight;
**characterized by**
determining means (DC) for effecting the determination of the selection of the eggs in such a manner that, until the number of selected eggs reaches a set number, an egg is selected as one of the predetermined target number of eggs if the weight of the egg fits within the predetermined permissible unit weight range and also that, after the number of selected eggs has reached said set number, a further egg is selected as another one of the predetermined target number of eggs if the weight of the further egg falls within said predetermined permissible unit weight range and if a subtotal weight resulting from addition of the weight of the further egg to the total weight of the previously selected eggs falls within a selective permissible weight range which becomes narrower with increase in the number of selected eggs; and
transferring means (TR) for transferring the selected eggs to a packaging container (P) based on the determination made by said determining means (DC).

5. An apparatus as defined in claim 4,
**characterized in that**
according to said determining means (DC), with selection of a further egg, a lower limit value of said selective permissible weight range increments, from an expected minimum added-up weight of the set number of selected eggs, by a value obtained by dividing a difference between a lower limit value of the target sum total weight range and the expected minimum added-up weight of the set number of selected eggs by a difference between the target number and the set number; and
with selection of the further egg, an upper limit value of said selective permissible weight range increments, from an expected maximum added-up weight of the set number of selected eggs, by a value obtained by dividing a difference between an upper limit value of the target sum total weight range and the expected maximum added-up weight of the set number of selected eggs by a difference between the target number and the set number.

6. An apparatus as defined in claim 4 or 5,
**characterized in that**
said determining means (DC) sets said set number as substantially to the half of said target number.

## Patentansprüche

1. Verfahren zum Selektieren von Eiern enthaltend die Schritte:
Selektieren einer vorbestimmten Zielanzahl von Eiern aus einer Vielzahl von Eiern, die zuvor individuell gewogen werden und in einer Reihe transportiert werden, in einer solchen Weise, dass das individuelle Gewicht eines jeden selektierten Eies innerhalb eines vorbestimmten zulässigen Einheitsgewichtsbereiches liegt und dass außerdem das Gesamtgewicht der Gruppe von selektierten Eiern innerhalb eines vorbestimmten zulässigen Gesamtsummengewichtbereiches liegt, der ein Zielsummengesamtgewicht enthält,
**dadurch gekennzeichnet**
**dass**, bis die Anzahl der selektierten Eier eine festgesetzte Anzahl erreicht, ein Ei selektiert wird als eines der vorbestimmten Zielanzahl von Eiern, falls das Gewicht des Eis innerhalb des vorbestimmten zulässigen Einheitsgewichtbereiches liegt und dass,
nachdem die Anzahl der selektierten Eier die genannte festgesetzte Anzahl erreicht hat, ein weiteres Ei selektiert wird als ein zusätzliches der vorbestimmten Zielanzahl von Eiern, falls das Gewicht des weiteren Eis innerhalb des vorbestimmten zulässigen Einheitsgewichtbereiches liegt und falls ein Zwischensummengewicht, das aus einer Addition des Gewichts des weiteren Eis zu dem Gesamtgewicht der zuvor selektierten Eier resultiert, innerhalb eines wählbaren zulässigen Gewichtsbereiches liegt, der enger wird mit Erhöhung der Zahl von selektierten Eiern.

2. Verfahren gemäß Anspruch 1,
**dadurch gekennzeichnet,**
**dass** sich mit der Selektion eines weiteren Eis ein unterer Grenzwert des genannten wählbaren zulässigen Gewichtsbereiches erhöht, von einem erwarteten minimalen aufaddierten Gewicht der festgesetzten Anzahl von selektierten Eiern um einen Wert, der erhalten wird durch Dividierung einer Differenz zwischen einem unteren Grenzwert des Zielsummengesamtgewichtsbereiches und dem erwarteten minimalen aufaddierten Gewicht der festgesetzten Anzahl von selektierten Eiern durch eine Differenz zwischen der Zielanzahl und der festgesetzten Anzahl, und
**dass** sich mit Selektion des weiteren Eis der obere Grenzwert des genannten wählbaren zulässigen Gewichtsbereiches erhöht, von einem erwarteten maximalen aufaddierten Gesamtgewicht der festgesetzten Anzahl von selektierten Eiern um einen Wert, der erhalten wird durch Dividierung einer Differenz zwischen einem oberen Grenzwert des Zielsummengesamtgewichtbereiches und dem erwarteten maximalen aufaddierten Gewicht der festgesetzten Anzahl von selektierten Eiern durch eine Differenz zwischen der Zielanzahl und der festgesetzten Anzahl.

3. Verfahren gemäß Anspruch 1 oder 2,
**dadurch gekennzeichnet,**
**dass** die festgesetzte Anzahl im wesentlichen auf die Hälfte der genannten Zielanzahl gesetzt wird.

4. Vorrichtung zum Selektieren von Eiern, wobei eine vorbestimmte Zielanzahl von Eiern aus einer Vielzahl von Eiern, die zuvor individuell gewogen wurden und in einer Reihe transportiert werden, in einer solchen Weise, dass das individuelle Gewicht von jedem selektierten Ei innerhalb eines vorbestimmten zulässigen Einheitsgewichtsbereiches liegen kann und dass außerdem das Gesamtgewicht der Gruppe von selektierten Eiern innerhalb eines vorbestimmten zulässigen Gesamtsummengewichtbereiches liegen kann, der ein Zielsummengesamtgewicht beinhaltet,
**gekennzeichnet durch**,
Bestimmungsmittel (DC) zur Bewirkung der Bestimmung der Selektion der Eier in einer solchen Weise, dass, bis die Anzahl der selektierten Eier eine festgesetzte Anzahl erreicht, ein Ei selektiert wird als eines der vorbestimmten Zielanzahl von Eiern, falls das Gewicht des Eis in den vorbestimmten zulässigen Einheitsgewichtbereich fällt und dass außerdem, nachdem die Anzahl der selektierten Eier die genannte festgesetzte Anzahl erreicht hat, ein zusätzliches Ei selektiert wird als ein weiteres der vorbestimmten Zielanzahl von Eiern, falls das Gewicht des weiteren Eis innerhalb des vorbestimmten zulässigen Einheitsgewichtbereiches liegt und
falls ein Zwischensummengewicht, das aus der Addition des Gewichtes des weiteren Eis zu dem Gesamtgewicht der zuvor selektierten Eier resultiert, innerhalb eines wählbaren zulässigen Gewichtsbereiches liegt, der enger wird mit der Erhöhung der Anzahl von selektierten Eiern und **durch**
Transfermitteln (TR) zum Tansferieren der selektierten Eier zu einem Verpackungsbehälter (P) basierend auf der Bestimmung, die **durch** die genannten Bestimmungsmittel (DC) gemacht wurde.

5. Vorrichtung gemäß Anspruch 4,
**dadurch gekennzeichnet,**
**dass** gemäß den genannten Bestimmungsmitteln (DC) mit der Selektion eines weiteren Eis sich ein niedrigerer Grenzwert des genannten wählbaren zulässigen Gewichtsbereiches erhöht, von einem erwarteten minimalen aufaddierten Gewicht der festgesetzten Anzahl selektierter Eier um einen Wert, der erhalten wird durch Dividierung einer Differenz zwischen einem unteren Grenzwert des Zielsummengesamtgewichtsbereiches und dem erwarteten minimalen aufaddierten Gewicht der festgesetzten Anzahl von selektierten Eiern durch eine Differenz zwischen der Zielanzahl und der festgesetzten Anzahl, und
**dass** sich mit Selektion eines weiteren Eis ein oberer Grenzwert des genannten wählbaren zulässigen Gewichtsbereiches erhöht, von einem erwarteten maximalen aufaddierten Gewicht der festgesetzten Anzahl von selektierten Eiern um einen Wert, der erhalten wird durch Dividierung einer Differenz zwischen einem oberen Grenzwert des Zielsummengesamtgewichtbereiches und dem erwarteten maximalen aufaddierten Gewicht der festgesetzten Anzahl von selektierten Eiern durch eine Differenz zwischen der Zielanzahl und der festgesetzten Anzahl.

6. Vorrichtung gemäß Anspruch 4 oder 5,
**dadurch gekennzeichnet,**
**dass** die Bestimmungsmittel (DC) die genannte festgesetzte Anzahl im wesentlichen auf die Hälfte der genannten Zielanzahl festsetzt.

## Revendications

1. Procédé de sélection d'oeufs comprenant les étapes consistant à :
sélectionner un nombre cible prédéterminé d'oeufs à partir d'une pluralité d'oeufs qui sont pesés individuellement à l'avance et qui sont transportés suivant une rangée, d'une manière telle que le poids individuel de chaque oeuf sélectionné se trouve à l'intérieur d'une plage de poids unitaire admissible prédéterminée et également que le poids total du groupe d'oeufs sélectionné se trouve à l'intérieur d'une plage de poids de la somme totale admissible prédéterminée comprenant un poids de somme totale cible,
**caractérisé en ce que**
jusqu'à ce que le nombre d'oeufs sélectionnés atteigne un nombre fixé, un oeuf est sélectionné en tant qu'oeuf parmi le nombre cible prédéterminé d'oeufs si le poids de l'oeuf se trouve à l'intérieur de la plage de poids unitaire admissible prédéterminée, et
après que le nombre d'oeufs sélectionnés a atteint ledit nombre fixé, un oeuf supplémentaire est sélectionné en tant qu'autre oeuf parmi le nombre cible prédéterminé d'oeufs si le poids de l'oeuf supplémentaire tombe à l'intérieur de ladite plage de poids unitaire admissible prédéterminée et si un poids du sous-total résultant de l'addition du poids de l'oeuf supplémentaire au poids total des oeufs précédemment sélectionnés tombe à l'intérieur d'une plage de poids admissible sélective qui devient plus étroite avec l'augmentation du nombre d'oeufs sélectionnés.

2. Procédé selon la revendication 1,
**caractérisé en ce que**
avec la sélection d'un oeuf supplémentaire, une valeur limite inférieure de ladite plage de poids admissible sélective augmente, à partir d'un poids ajouté minimum attendu du nombre fixé d'oeufs sélectionnés, d'une valeur obtenue en divisant une différence entre une valeur limite inférieure de la plage de poids de somme totale cible et du poids ajouté minimum attendu du nombre fixé d'oeufs sélectionnés par une différence entre le nombre cible et le nombre fixé, et
avec la sélection de l'oeuf supplémentaire, une valeur limite supérieure de ladite plage de poids admissible sélective augmente, depuis un poids ajouté maximum attendu du nombre fixé d'oeufs sélectionnés, d'une valeur obtenue en divisant une différence entre une valeur limite supérieure de la plage de poids de somme totale cible et le poids ajouté maximum attendu du nombre fixé d'oeufs sélectionnés par une différence entre le nombre cible et le nombre fixé.

3. Procédé selon la revendication 1 ou 2,
**caractérisé en ce que**
ledit nombre fixé est fixé pratiquement à la moitié dudit nombre cible.

4. Dispositif destiné à sélectionner des oeufs, dans lequel un nombre cible prédéterminé d'oeufs à partir d'une pluralité d'oeufs qui sont pesés individuellement à l'avance et qui sont transportés sur une rangée, d'une manière telle que le poids individuel de chaque oeuf sélectionné puisse se trouver à l'intérieur d'une plage de poids unitaire admissible prédéterminée et également que le poids total du groupe d'oeufs sélectionnés puisse se trouver à l'intérieur d'une plage de poids de somme totale admissible prédéterminée incluant un poids de somme totale cible,
**caractérisé par**
un moyen de détermination (DC) destiné à effectuer la détermination de la sélection des oeufs d'une manière telle que, jusqu'à ce que le nombre d'oeufs sélectionnés atteigne un nombre fixé, un oeuf est sélectionné en tant qu'un parmi le nombre cible prédéterminé d'oeufs si le poids de l'oeuf se trouve à l'intérieur de la plage de poids unitaire admissible prédéterminée et également que, après que le nombre d'oeufs sélectionnés a atteint ledit nombre fixé, un oeuf supplémentaire soit sélectionné en tant qu'autre oeuf parmi le nombre cible prédéterminé d'oeufs si le poids de l'oeuf supplémentaire tombe à l'intérieur de ladite plage de poids unitaire admissible prédéterminée et si un poids de sous-total résultant de l'addition du poids de l'oeuf supplémentaire au poids total des oeufs sélectionnés précédemment tombe à l'intérieur d'une plage de poids admissible sélective qui devient plus étroite avec une augmentation du nombre d'oeufs sélectionnés, et
un moyen de transfert (TR) destiné à transférer les oeufs sélectionnés vers un récipient de conditionnement (P) sur la base de la détermination réalisée par ledit moyen de détermination (DC).

5. Dispositif selon la revendication 4,
**caractérisé en ce que**
conformément audit moyen de détermination (DC), avec la sélection d'un oeuf supplémentaire, une valeur limite inférieure de ladite plage de poids admissible sélective augmente, depuis un poids ajouté minimum attendu du nombre fixé d'oeufs sélectionnés, d'une valeur obtenue en divisant une différence entre une valeur limitée inférieure de la plage de poids de somme totale cible et le poids ajouté minimum attendu de l'ensemble fixé d'oeufs sélectionnés par une différence entre le nombre cible et le nombre fixé, et
avec la sélection de l'oeuf supplémentaire, une valeur limite supérieure de ladite plage de poids admissible sélective augmente, depuis un poids ajouté maximum attendu du nombre fixé d'oeufs sélectionnés, d'une valeur obtenue en divisant une différence entre une valeur limite supérieure de la plage de poids de somme totale cible et le poids ajouté maximum attendu du nombre fixé d'oeufs sélectionnés par une différence entre le nombre cible et le nombre fixé.

6. Dispositif selon la revendication 4 ou 5,
**caractérisé en ce que**
ledit moyen de détermination (DC) fixe ledit nombre fixé pratiquement à la moitié dudit nombre cible.
